**Europäisches Patentamt**

**European Patent Office**

**Office européen des brevets**

(11) Veröffentlichungsnummer: **0 166 899 B1**

# EUROPÄISCHE PATENTSCHRIFT

(45) Veröffentlichungstag der Patentschrift: **27.02.91**

(51) Int. Cl.5: **G01N 21/35**, G01N 33/44

(21) Anmeldenummer: **85105146.6**

(22) Anmeldetag: **26.04.85**

(54) **Verfahren zur Untersuchung von Kunststoffkörpern.**

(30) Priorität: **04.05.84 DE 3416594**

(43) Veröffentlichungstag der Anmeldung:
**08.01.86 Patentblatt 86/02**

(45) Bekanntmachung des Hinweises auf die
Patenterteilung:
**27.02.91 Patentblatt 91/09**

(84) Benannte Vertragsstaaten:
**AT BE CH DE FR GB IT LI LU NL SE**

(56) Entgegenhaltungen:
**EP-A- 0 068 086**
**EP-A- 0 070 610**
**NL-A- 8 103 140**

**BRAUWELT, Nr. 7, 15. Februar 1979, Seiten
183-188; O. HAHMANN et al.: "18 Jahre Einsatzerfahrungen mit Flaschenkästen aus Polyethylen"**

**R. NITSCHE et al.: "Kunststoffe", Band II,
1961, Seiten 266-269**

**K. THINIUS: "Stabilisierung und Alterung von
Plastwerkstoffen", Band II, Abschnitt 4.2.2.1.,
1971, Seiten 116-131, Verlag Chemie GmbH**

**PLASTICS ENGINEERING-SPE JOURNAL,
Band 28, Juli 1972, Seiten 19-24; F.H. WINS-
LOW et al.: "Polymers under the weather"**

(73) Patentinhaber: **ALEXANDER SCHOELLER &
CO. AG**
**Hölzliwiesenstrasse 9 Volketswil Post**
**CH-8603 Schwerzenbach(CH)**

(72) Erfinder: **Schoeller, Martin, Dipl.-Ing.**
**Haus Balaster**
**CH-7524 Zuoz(CH)**
Erfinder: **Schoeller, Christoph, Dipl.-Ing.**
**Haus Balaster**
**CH-7524 Zuoz(CH)**

(74) Vertreter: **Dr. Elisabeth Jung Dr. Jürgen Schirdewahn Dipl.-Ing. Claus Gernhardt**
**P.O. Box 40 14 68 Clemensstrasse 30**
**D-8000 München 40(DE)**

## Beschreibung

Flaschenkästen, aber auch andere Spritzgußgroßteile oder sonstige Untersuchungskörper aus Kunststoff, werden nicht nur aus Neuware, also aus sozusagen "jungfräulichem" oder "erstverspritztem",fabrikfrischem Kunststoff, sondern zum Teil auch aus Regenerat hergestellt. Im Gegensatz zu Kunststoff aus Regenerat wird Kunststoff aus Neuware auch unregenerierter Kunststoff genannt. Flaschenkästen werden in der Bundesrepublik Deutschland fast ausschließlich aus Polyäthylen (PE), und zwar meist aus Niederdruck-Polyäthylen (HDPE), hergestellt. Es ergibt sich dabei, daß Flaschenkästen aus Regenerat denjenigen aus Neuware praktisch gleichwertig sein können, wenn bestimmte Bedingungen, wie bezüglich des Oxidationsgrades, eingehalten werden. Völlig versprödete Altkästen erweisen sich zur Gewinnung von Regenerat als nicht geeignet. Regenerat wird meist aus noch geeigneten Altkästen unter Zusetzung eines gewissen Anteils von Neuware und gegebenenfalls Ausscheidung von bestimmten Anteilen der Kunststoffmasse des Altkastens hergestellt.

Flaschenkästen aus Regenerat unterscheiden sich daher im maße ihrer inneren Oxidation. Nähere Einzelheiten ergeben sich hinsichtlich Flaschenkästen aus der Veröffentlichung "Flaschenkästen aus Neuware und Flaschenkästen aus Regenerat" von Dr. Kremkow, Verpackungsprüfstelle Vertragslaboratorium des Deutschen Brauerbundes e.V. an der Versuchs- und Lehranstalt der Brauerei in Berlin, in "Das Erfrischungsgetränk - Mineralwasser-Zeitung", S. 262 bis 267, gemäß anliegender Kopie.

Die Erfindung befaßt sich insbesondere mit derartigen Flaschentransportkästen, allgemeiner aber auch mit sonstigen Spritzgußgroßteilen aus Kunststoff, z.B. Kunststoffpaletten, und allgemein auch mit sonstigen Untersuchungskörpern aus Kunststoff, soweit eine vergleichbare Problematik gegeben ist.

Aus dem Rundschreiben T 22 / E 2 - 1980 des "DEUTSCHER BRAUERBUND E.V." vom 8. April 1980, insbesondere S. 2, 2. vollst. Abs., ergibt sich, daß inzwischen schon labormäßig Untersuchungsmethoden für die einwandfreie Differenzierung zwischen brauchbarem und mangelhaftem Regenerat von der Verpackungsprüfstelle des "DEUTSCHER BRAUERBUND E.V." entwickelt seien, während aber eine Differenzierung zwischen Neuware und brauchbarem Regenerat erheblich schwieriger sei und gegenwärtig noch nicht ausgeführt werden könne. Dasselbe Bild ergab sich noch auf einer Tagung der Gütegemeinschaft Transport- und Lagerbehälter im Qualitätsverband Kunststofferzeugnisse e.V., Gütegruppe Flaschenkasten, am 02.03.1984. Bei dieser Tagung hat sogar der Hersteller von Vorrichtungen gemäß der EP-A1 0 070 610, welche zur Alterungsmessung an Flaschenkästen und Aussortierung nicht mehr geeigneter Flaschenkästen bestimmt ist, einräumen müssen, zwischen Flaschenkästen aus Neuware und Flaschenkästen aus Regenerat nicht unterscheiden zu können.

In der eingangs erwähnten Veröffentlichung von Kremkow, S. 262, 3. Sp., mittl. Abs., ist die schon jahrelang den Brauereifachleuten bekannte Erkenntnis zu entnehmen, daß bei einem Flaschenkasten besonders die Griffleiste gefährdet ist, die am oberen Rand des Flaschenkastens oberhalb von seitlichen Öffnungen in der Flaschenkastenwand ausgebildet ist und wegen der Verbindung über nur einige vertikale Stege besonders leicht abbrechen kann. Man hat daher auch bei mechanischen Messungen des Alterungszustandes von Flaschenkästen die Griffleiste des Flaschenkastens für die Untersuchung des Alterungszustandes gewählt (DE-PS 29 22 535). Dementsprechend wird nach der EP-A1 0 070 610 die Griffleiste ebenfalls für Alterungsuntersuchungen zugrundegelegt, jedoch mittels eines infrarot-spektrometrischen Transmissions-Meßverfahrens. Es wird dabei die Hypothese zugrundegelegt, daß für die Alterung von Flaschenkästen Oberflächenerscheinungen vernachlässigt werden könnten, da in der Kunststoffoberfläche nur in einem relativ schnellen Prozeß freie Radikale (Ester) entstehen würden, während als für die Alterung maßgeblicher Prozeß in der Tiefe der Flaschenkastenwand in wesentlich längeren Zeiträumen als an der Oberfläche CO-Gruppen (Carbonyl-Gruppen) ständig zunehmend entstünden (EP-A1 0 070 610, S. 3, Z. 11 bis 24). Diese Hypothese stützt sich darauf, daß mechanische Festigkeitseigenschaften des Kunststoffes in erster Linie von inneren Eigenschaften des Kunststoffes abhängen müßten und erscheint daher durchaus plausibel. Dementsprechend wird nach der EP-A1 0 070 610 die Griffleiste des Flaschenkastens infrarot-spektrometrisch durchleuchtet und die Absorption an der Carbonyl-Gruppen-Bande gemessen, die etwa bei Wellenzahlen zwischen 1690 und 1740 cm$^{-1}$ mit Maximum etwa bei 1720 cm$^{-1}$ liegt. Dieselben Carbonyl-Gruppen liegen jedoch in der Tiefe der Flaschenkastenwand bei Flaschenkästen aus Regenerat von vornherein vor, und zwar in mehr oder minder großer Konzentration je nach der Art des Regenerats. Das Meßverfahren der EP-A1 0 070 610 ist daher schon seinem Denkansatz nach nicht in der Lage, zwischen gealterten Flaschenkästen aus Neuware und neuen Flaschenkästen aus Regenerat zu unterscheiden. Auch war die Durchführung infrarot-spektrometrischer Transmissions-Meßverfahren an Folien oder auch Platten aus Kunststoff schon bekannt aus der Monographie von R. Nitsche und K. Wolf: Kunststoffe, Springerverlag, Berlin/Göttingen/Heidelberg, 1961, Bd. 2 (Praktische Kunststoffprüfung), S. 266 bis 269, insbes. S. 268.

Andererseits ist es schon aus der Zeit des Zweiten Weltkrieges bekannt, daß die Eigenschaften von Polyäthylen und vergleichbaren anderen Kunststoffen durch infrarot-spektrometrische Meßmethoden festgestellt werden können (vgl. Fußnote auf S. 1 des Artikels "The Infra-Red Spectrum of Ethylene Polymers" von L.H. Cross u.a. in "Discussion Faraday Society", 1950, S. 235 bis 245). In dieser Veröffentlichung wird auf S. 243 die Oxidation durch Wärmeeinwirkung und auf S. 244, Mitte, die Photo-Oxidation abgehandelt, mit dem Ergebnis, daß die Konzentration von Carbonyl-Gruppen unter Berücksichtigung der Dicke des betreffenden Untersuchungskörpers quantitativ infrarot-spektrometrisch im Bereich der Carbonyl-Gruppen-Bande gemessen werden könne.

Spätestens schon seit dem Artikel von Rugg u.a. mit dem Titel "Infrared Spectrophotometric Studies on Polyethylene, II. Oxidation" in "Journal of Polymer Science", Vol. XIII, pages 535 to 547 (1954), insbes. S. 541, untere Hälfte, ist es bereits bekannt, daß sowohl Oxidation unter Wärmeeinfluß als auch Photo-Oxidation zu einer Carbonyl-Gruppen-Bildung im Kunststoff führen, die jeweils infrarot-spektrometrisch als Transmissions-Absorption im Bereich der Carbonyl-Gruppen-Bande gemessen werden kann. Die entsprechenden Absorptionen unterscheiden sich dabei lediglich quantitativ. Besonders bezüglich der Photo-Oxidation wird hervorgehoben, daß sie zu einer Brüchigkeit des Untersuchungskörpers führt. Als Untersuchungskörper wurden Folien mit einer Stärke von 0,8 mm verwendet. Entsprechende Erkenntnisse fanden dann auch schon spätestens Anfang der Siebziger Jahre Eingang im Lehrbuch-Wissen, siehe die Monographie "Stabilisierung und Alterung von Plastwerkstoffen", Bd. II, "Alterung der Plastwerkstoffe", von Prof. Thinius, Verlag Chemie GmbH 1971, S. 116 bis 131, insbes. S. 126 und 127. Auch in dem Norm-Entwurf der deutschen DIN-Norm 53 383, Teil 2, März 1979 (Einsprüche bis 31. Juli 1979), welche die Prüfung der Oxidationsstabilität von Kunststoffen, insbes. HDPE, durch Ofenalterung betrifft, ist bereits die Messung der für die Oxidationsstabilitätszeit maßgeblichen Menge an vom HDPE-Formstoff chemisch gebundenem Sauerstoff durch Aufnahme der Transmissions-Absorptions-Werte im Bereich der Carbonyl-Gruppen-Bande bei einer Wellenzahl von 1720 cm$^{-1}$ vorgeschrieben, wobei die zugrundegelegten Probekörper eine Dicke von 0,3 mm haben sollen.

Aus den genannten Literaturstellen ist ferner zu entnehmen, daß die Alterung von Kunststoffen primär von der Photo-Oxidation der Sonneneinstrahlung und nur sekundär von durch Wärmeeinwirkung entstehender Oxidation abhängt. Die Einwirkung der Photo-Oxidation hat jedoch nur eine relativ geringe Eindringtiefe. Dies ergibt sich aus dem Artikel "Polymers Under the Weather" von Winslow u.a. in SPE Journal, July 1972 - Vol. 28, S. 19 bis 24, insbes. S. 21, rechte Sp. Danach nimmt die Oxidationsrate innerhalb 200 Stunden bei Polyäthylen-Folien mit einer Dicke von 0,05 mm noch linear, bei Dicken von 0,13 bis 1,3 mm jedoch immer abgeflachter zu, mit der Schlußfolgerung, daß es sich bei der Photo-Oxidation primär um einen Oberflächeneffekt handele. Entsprechende Ergebnisse wurden für Polypropylen erzielt. Demzufolge wurden gemäß der hüls-Publikation von Dr. Hahnmann u.a. "18 Jahre Einsatzerfahrungen mit Flaschenkästen aus Polyäthylen" in "Brauwelt" 7/1979, S. 183 bis 188, Sonderdruck: 5163 der hüls Chemische Werke Hüls Aktiengesellschaft, D-4370 Marl 1, S. 1 bis 7, insbes. S. 4, rechte Sp., zur Messung des Alterungszustandes von Flaschenkästen diesen Oberflächenproben aus einer Oberflächenschicht von 0,02 mm entnommen und infrarot-spektrometrisch auf Carbonyl-Gruppen untersucht. Die EP-A1 0 070 610 geht gattungsgemäß von einem solchen Stand der Technik aus, verwirft ihn jedoch als für die Messung des tatsächlichen Alterungszustandes nicht geeignet (S. 2, Z. 3 bis 15, und S. 3, Z. 7 bis 24).

Unter Überwindung dieses Vorurteils geht die Erfindung von folgender alternativen Hypothese aus:

Bezüglich Kunststoffs als Neuware wird angenommen, daß keine oder doch eine in erster Näherung vernachlässigbare Konzentration von Carbonyl-Gruppen in Kunststoff vorliegt. Bezüglich Kunststoffs aus Regenerat wird angenommen, daß eine bestimmte ortsunabhängige Konzentration an Carbonyl-Gruppen über die ganze Tiefe des Kunststoffs verteilt ist. In beiden Fällen, ob Neuware oder Regenerat, wird ferner angenommen, daß die alterungsspezifischen Vorgänge nur in einer Oberflächenschicht erfolgen, deren Dicke klein gegenüber der Probendicke ist und sich beispielsweise in einer zunehmenden Versprödung und damit auch Kerbanfälligkeit unter Photo-Oxidationseinflüssen des Sonnenlichts äußern. Diese Hypothese steht in guter Übereinstimmung mit eigenen Erfahrungen der Anmelderin an Flaschenkästen.

Bei strenger Gültigkeit dieser Hypothese sind bei Untersuchungskörpern aus Neuware infrarot-spektrometrisch gemessene Werte der Transmissions-Absorption im Bereich der Carbonyl-Gruppen-Bande unabhängig von der Dicke des durchstrahlten Untersuchungskörpers. Das Maß der gemessenen Absorption ist dabei ausschließlich durch die Oberflächen-Oxidation bedingt und daher als Kriterium für den Alterungszustand des Untersuchungskörpers verwendbar. Anders als bei der EP-A1 0 070 610 wird hierbei also der Alterungseffekt auf einen Oberflächeneffekt und nicht auf einen integralen Tiefeneffekt zurückgeführt.

Auch bei Untersuchungskörpern aus Regenerat erfolgt an der Oberfläche eine Transmissions-Absorption im Bereich der Carbonyl-Gruppen-Bande in unterschiedlicher Stärke je nach dem Alterungszustand des Untersuchungskörpers. Darüber hinaus sind jedoch infolge der im Regenerat in der Tiefe gleichmäßig eingelagerten Carbonyl-Gruppen nach dem Beer-Lambert'schen Gesetz die dort zusätzlich auftretenden

EP 0 166 899 B1

Transmissions-Absorptionswerte proportional der Dickenänderung linear zunehmend.

In der Praxis mögen die Verhältnisse gemäß der geschilderten, der Erfindung zugrundeliegenden Hypothese nicht so rein vorliegen, wie dies soeben besprochen wurde.

Trotzdem erscheint es gerechtfertigt, die bisher noch ungelöste Aufgabe, bei gleichartigen Kunststoffkörpern, insbesondere aus Polyäthylen oder Polypropylen, die Eigenschaften zu messen,
- ob sie aus unregeneriertem oder regeneriertem Kunststoff bestehen,
- wie hoch bei regeneriertem Kunststoff der Grad der in der Tiefe verteilten Oxidation ist, und/oder
- wie hoch der Grad der Oberflächenoxidation ist, unabhängig davon, ob die Kunststoffkörper aus unregeneriertem oder regeneriertem Kunststoff bestehen, dadurch zu lösen, daß die IR-Transmission im Bereich der Carbonylgruppenbande bei mindestens zwei verschiedenen Dicken des Kunststoffs gemessen wird. Dabei sollen als Äquivalente auch Messungen solcher Neben-Bande mit eingeschlossen sein, welche vergleichbare Meßergebnisse hervorbringen. Einige mögliche derartige Neben-Bande sind in der erwähnten vorveröffentlichten Literatur angegeben. Von besonderem Interesse sind dabei solche Kunststoffe aus Niederdruck-Polyäthylen oder Niederdruck-Polypropylen, wie sie regelmäßig bei Flaschenkästen Verwendung finden.

Bei vollständiger Auswertung der erfindungsgemäßen Messung kann man alle drei gesuchten Eigenschaften bestimmen. So ist bei Kunststoffkörpern aus Neuware bzw. unregeneriertem Kunststoff die IR-Transmission dickenunabhängig, jedoch bei Kunststoffkörpern aus Regenerat dickenabhängig, nämlich proportional zur Dickenänderung. Bei Kunststoffkörpern aus Regenerat ist dabei der Proportionalitätsfaktor, mit dem sich die IR-Transmission mit der Dickenänderung ändert, bzw. die Steilheit der IR-Transmissionskurve in Abhängigkeit von der Dickenänderung, das Maß für den Grad, der in der Tiefe des Kunststoffkörpers verteilten Oxidation und damit ein Maß für den Regenerationsgrad des Kunststoffs. Unabhängig davon, ob die Kunststoffkörper aus unregeneriertem oder regeneriertem Kunststoff bestehen, ergibt sich ferner der Grad der Oberflächenoxidation aus dem Extrapolationswert der gemessenen IR-Transmissionen auf die Dicke Null bzw. fast Null. Es versteht sich, daß man sich bei praktischen Messungen auf die Bestimmung nur von zwei oder auch nur von einer der genannten Eigenschaften beschränken kann.

Die Messungen der IR-Transmission bei verschiedenen Dicken können grundsätzlich sukzessive durchgeführt werden; vorgezogen wird jedoch eine simultane Messung gemäß Anspruch 2. Im Regelfall wird man dabei mit einer einmaligen Durchstrahlung jeder der unterschiedlich dicken Stellen des Kunststoffkörpers auskommen; im Sonderfall, insbesondere bei Messung an relativ dünnen Stellen, wird man jedoch auch eine mehrmalige Durchstrahlung derselben Stelle des Kunststoffkörpers in Betracht ziehen.

In einer Weiterbildungsmöglichkeit der Erfindung nach Anspruch 6 kann man sogar zugleich mindestens zwei Wände des Untersuchungskörpers durchstrahlen und so den Dicken-Meßbereich noch spreizen, um noch besser zwischen Oberflächeneffekten und Tiefeneffekten unterscheiden zu können.

Die gemäß der Erfindung gewonnenen Meßwerte der Transmissions-Absorption im Bereich der Carbonyl-Gruppen-Bande und gegebenenfalls der PE-Bande oder einer vergleichbaren Bande bei verschiedenen Dicken lassen sich also nicht nur zur Unterscheidung von Neuware und regenerierter Ware oder verschiedenen Arten von regenerierter Ware nutzen, sondern erstmals auch zur Festlegung eines Alterungskriteriums des Untersuchungskörpers unabhängig davon, ob dieser aus Neuware oder regenerierter Ware besteht. Hierzu dient die Bestimmung des Grades der Oberflächenoxidation. Für den Fall eines Untersuchungskörpers aus Neuware und unter der Annahme, daß die der Erfindung zugrundegelegte Hypothese streng stimmt, erhält man dabei wegen der Dicken-Unabhängigkeit der Messung dieselben Transmissions-Absorptions-Werte bei der Carbonyl-Gruppen-Bande wie bei der integrierenden Messung der Carbonyl-Gruppen-Bande mittels Durchstrahlung mit einem einzigen Meßstrahl. Für Untersuchungskörper aus regeneriertem Kunststoff kann man überhaupt erstmals ein quantitatives Kriterium für oberflächliche Alterung angeben. Es ist dabei sogar nicht mehr nötig, die ermittelte Eigenschaft, ob es sich um einen Untersuchungskörper aus Neuware oder in unterschiedlichem Maß oxidierter regenerierter Ware handelt, direkt zur Kenntnis zu nehmen; man kann sogar die betreffenden Meßwerte unmittelbar weiterverarbeiten und den Grad der oberflächlichen Alterung angeben, ohne sich noch Rechenschaft darüber ablegen zu müssen, ob und in welchem Maße der Kunststoff des Untersuchungskörpers regeneriert ist. Dies ermöglicht also überhaupt erstmals eine echte Alterungsmessung von gleichartigen Kunststoffkörpern für alle die Fälle, in denen mit dem Auftreten von Kunststoffkörpern aus Regenerat gerechnet werden muß. Dies ist beispielsweise in der Bundesrepublik Deutschland bei Flaschenkästen regelmäßig der Fall

Wegen der Empfindlichkeit der erfindungsgemäßen Messung kann man in manchen Fällen darauf verzichten, die genauen Werte der unterschiedlichen Dicke des jeweils zugrundeliegenden Untersuchungskörpers zu kennen; denn gemäß der genannten, der Erfindung zugrundeliegenden Hypothese ist ja bei Neuware das Meßergebnis dickenunabhängig und bei regenerierter Ware proportional zur Dickenänderung, ein Unterschied, der auch bei nicht oder nicht genau bekannter Größe der unterschiedlichen Dickenwerte

4

ohne weiteres schon bei zwei Messungen erkennbar ist, wenn der Dickenunterschied hinreichend signifikant groß ist. Entsprechend kann man auch bei nur ungenau bekannten Dickenwerten die Steilheit der IR-Transmission in Abhängigkeit von der Dickenänderung bzw. den Extrapolationswert der IR-Transmission auf die Dicke Null oft noch hinreichend genau abschätzen. Ähnliches sollte auch in vielen praktischen Fällen weiterhin gelten, selbst wenn von einem nicht idealen Vorliegen der der Erfindung zugrundeliegenden Hypothese ausgegangen werden muß. Eine größere Sicherheit erhält man jedoch in jedem Falle bei genaueren Werten der zugrundeliegenden Dicken des jeweils durchstrahlten Untersuchungskörpers. Diese Dicken kann man auf irgendeine konventionelle Weise messen. Bevorzugt wird es jedoch, gemäß Anspruch 3 oder 4 die betreffende Dicke selbst auch infrarot-spektrometrisch zu messen, und zwar entweder an derselben oder nahe benachbarten Stelle, an der die Transmissions-Absorptions-Messung der Carbonyl-Gruppen-Bande jeweils erfolgt, oder an einer anderen Stelle, bei der gleiche oder vergleichbare Dicke vorausgesetzt werden kann. Diese Dickenmessung kann zu-gleich mit der Messung der Carbonyl-Gruppen-Bande oder auch sukzessive vorher oder nachher erfolgen; zur infrarot-spektrometrischen Transmissions-Absorptions-Messung eignet sich dabei jede Bande, welche ganz oder weitgehend unabhängig vom Vorhandensein von Carbonyl-Gruppen, aber nach dem Beer-Lambert'schen Gesetz proportional der Konzentration typischer Gruppen des betreffenden Kunststoffs sowie ferner proportional der durchstrahlten Dicke (also proportional der durchstrahlten Kunststoffmasse) ist. Im Falle von Polyäthylen besonders geeignet ist dabei die sog. PE-Bande mit den charakteristischen Wellenzahlen 1820 - 1900 - 2000 $cm^{-1}$, bei Polypropylen die entsprechende PP-Bande. Es kommt jedoch wiederum auch jede andere vergleichbare Neben-Bande in Frage.

Es ist grundsätzlich möglich, die erfindungsgemäßen Messungen, gegebenenfalls sogar nur mit einem Meßstrahl, an verschiedenen gleichartigen Kunststoffkörpern an Stellen unterschiedlicher Dicke durchzuführen. Vorgezogen wird jedoch, gemäß Anspruch 5 die bei verschiedenen Dicken vorgenommenen Messungen an Stellen verschiedener Dicke desselben Kunststoffkörpers vorzunehmen.

Es ist an sich bekannt (EP-A1 0 070 610), bei verschiedenen Spektrallinien oder -banden zu messen. Dabei wird jedoch nur eine Kalibrierung der Messung in Betracht gezogen, indem in Nachbarschaft der Carbonyl-Gruppen-Bande das Grundniveau der spektralen Absorptions-Verteilung bestimmt wird, um den quantitativen Überstand der gemessenen Carbonyl-Gruppe-Bande über die Grundlinie des Spektrums durch Differenzbildung messen zu können. Eine Messung von Carbonyl-Gruppen-Banden an mehr als einer Stelle des Flaschenkastens oder auch die Verwendung von dessen im Spektrum sogar eingezeichneter PE-Bande zur Dickenmessung ist nicht in Betracht gezogen.

Als Untersuchungskörper kommen gemäß Anspruch 7 außer Flaschenkästen insbesondere auch andere Spritzgußgroßteile aus Niederdruck-Polyäthylen und anderen vergleichbaren Kunststoffen, beispielsweise auch Niederdruck-Polypropylen, in Frage. Dies ergibt sich insbesondere aus der 11. Seite, rechte Sp. 3. und 4. Abs. von unten, eines 12-seitigen Sonderdruckes aus dem KUNSTSTOFFBERATER, Heft 8 bis 10/1968, Frankfurt/Main, mit dem Titel "Probleme bei Spritzgußgroßteilen aus Niederdruck-Polyäthylen, demonstriert am Flaschentransportkasten" von Dr. Hahmann u.a., Chemische Werke Hüls AG. Danach erfolgt eine physikalische Alterung durch zunehmende Versprödung durch chemischen Abbau, der jedoch nur an der Oberfläche stattfinde und sich in einer feinen Rißbildung an der Oberfläche äußere, die durch ihre außerordentlich hohe Kerbwirkung bei Beanspruchung zum Bruch der Fertigteile führe. Als Kriterium hierfür wird im Infrarot-Spektrum ein Carbonyl-Gruppen-Gehalt von mehr als 0,3 % angegeben.

Die Erfindung erstreckt sich ferner auch noch auf alle sonstigen Untersuchungskörper aus Kunststoff, bei denen zur Unterscheidung von Neuware und regeneriertem oder in verschiedenem Maße regeneriertem Kunststoff dickenabhängig gemessen werden kann bzw. zur Feststellung des Alterungszustandes an der dicken-abhängigen Meßkurve des Carbonyl-Gruppen-Bandes noch zwischen Oberflächeneffekten und integrierten Tiefeneffekten unterschieden werden kann.

Schon in ihrem allgemeinsten Rahmen ermöglicht es die Erfindung, die nach ihrem Material (Neuware und Regenerat) unterschiedenen Untersuchungskörper zu klassifizieren und gegebenenfalls zu sortieren, also zu klassieren. Von besonderer Bedeutung ist jedoch eine Klassifizierung und gegebenenfalls Sortierung nach dem Alterungszustand im Sinne der Verwendung des erfindungsgemäßen Verfahrens nach Anspruch 9, wobei der Alterungszustand nach Anspruch 1, dritte Alternative, gemessen werden kann. Ein mögliches Vorbild für eine solche berührungsfreie Klassifizierung und gegebenenfalls Sortierung zeigt beispielsweise der Artikel von Harris in "Automation", Januar 1970, S. 53 bis 57, wo im letzten Abschnitt auf S. 57 und im Abstract auf S. 53 über die zunehmende Einführung auch in der kunststoffverarbeitenden Industrie von berührungslos arbeitenden spektrometrischen Sortierverfahren von auf einer Transferstrecke ankommenden Untersuchungskörpern anhand von Polyäthylen-Pellets berichtet wird (vgl. auch GB-A-2 060 166, US-PS 3 448 268 und FR-PS 1 501 766). Die in den genannten Fällen mit Reflexions-Spektrometrie arbeitenden Verfahren können dabei sinngemäß unter Verwendung von Meßkammern für Transmissions-Spektrometrie durchgeführt werden, welche der eingangs erörterte Stand der Technik als bekannt voraussetzt und von denen eine mögliche Ausführung in der "Revue Générale de l'Electricité", Vol. 88, No. 9, September 1979, Paris (FR), P. Laurenson et al: "Photovieillissement et environnement. Nouveau dispositif de photovieillissement accéléré et élaboration d'isolants de couleurs photostables", S. 685 bis 689, beschrieben ist.

Anspruch 10 zeigt sogar erstmalig einen Weg auf, mit denselben berührungslosen Klassifizierungs- und gegebenenfalls Sortierverfahren gealterte Kunststoffkästen oder andere gleichartige Kunststoffkörper danach zu unterscheiden und gegebenenfalls auszuwählen, ob sie zur Weiterverwendung als Regenerat noch gebraucht werden können oder endgültig ausgeschieden werden müssen. Dadurch wird erstmalig eine bequeme Möglichkeit erhalten, durch ein ganz einfaches berührungsloses und dementsprechend auch zerstörungsfreies Testverfahren zuverlässig geeignetes Regenerat zurückzugewinnen und so in ganz erheblichem Maße den Verbrauch an Kunststoff-Neuware einzuschränken.

Hierdurch entsteht zum ersten Mal die Möglichkeit, ein homogenes Ausgangsmaterial zum Regenerieren zu erhalten und einen aus solchem Regenerat hergestellten Kunststoffkörper auch später erkennen und passieren zu lassen. Diese Erfindung ermöglicht somit erstmalig auch ein qualitätsgesichertes Recycling mit den sinngemäßen Verfahrensschritten des (1) Sortierens, (2) Homogenisierens und Restabilisierens zur Regeneratgewinnung, (3) neuen optimierten Design und (4) Produzierens von Kunststoffartikeln aus Regenerat, d.h. aus im Material angepaßtem, z.B. nach bestimmten Rezepturen aus Regenerat und Neuware gemischtem Kunststoff, wobei die Erfindung zugleich Werte zur Feststellung der Restlebenserwartung der aus Regenerat gefertigen Kunststoffartikel zur Verfügung stellt. Dies ermöglicht insbesondere modernste Herstellungsweisen von Kunststoffartikeln aus Regenerat im Wege einer CAE-Optimierung des Formwerkzeugs und einer CAD-Optimierung des Produkts (CAE = Computer Aided Engineering, CAD = Computer Aided Design).

In dem bevorzugten Anwendungsfall des Flaschenkastens kann man, wie schon gesagt, beispielsweise als eine Meßstelle eine nicht gegen Sonneneinstrahlung abgeschattete Stelle des Griffrandes wählen, der beispielsweise 2,8 bis 4,0 mm stark ist, und als zweite Meßstelle einen nicht abgeschatteten unteren Bereich der Flaschenkastenwand, der beispielsweise zwischen 1,8 und 2,4 mm stark ist. Man kann aber auch alle anderen Meßstellen eines Untersuchungskörpers wählen, die gegen Sonneneinstrahlung nicht abgeschattet oder aber vergleichbar abgeschattet sind und die unterschiedliche Dicke haben. Dann ist die Schädigung (Alterung) an der Oberfläche als gleich oder mindestens vergleichbar anzusehen. Bei Flaschenkästen könnte man als dicke Stellen beispielsweise auch eine obere Randkante oder die Ecken der Flaschenkästen in Betracht ziehen (vgl. Anspruch 8).

Die Erfindung betrifft auch Vorrichtungen nach den Ansprüchen 11 bis 13 zum Ausführen des erfindungsgemäßen Verfahrens, und zwar im Rahmen der bevorzugten Verwendungen nach den Ansprüchen 7 oder 8.

Die Erfindung wird im folgenden anhand schematischer Zeichnungen an einer prinzipiellen Begründung des der Erfindung zugrundeliegenden Konzepts und an einem Ausführungsbeispiel noch näher erläutert. Es zeigen:

| | |
|---|---|
| Fig. 1a und 1b | zwei Wandausschnitte verschiedener Dicke eines Untersuchungskörpers aus Neuware; |
| Fig. 2a und 2b | zwei den Fig. 1a und 1b entsprechende Ausschnitte eines Untersuchungskörpers aus Regenerat; |
| Fig. 3 | ein Diagramm zur Darstellung der Abhängigkeit gemessener Transmissions-Absorptions-Werte im Bereich der Carbonyl-Gruppen-Bande in Abhängigkeit von der Dicke des Untersuchungskörpers, |
| Fig. 3a und 3b | bestimmte Varianten des Diagramms nach Fig. 3, und |
| Fig. 4 | eine Seitenansicht einer Vorrichtung zum Ausführen des erfindungsgemäßen Verfahrens. |

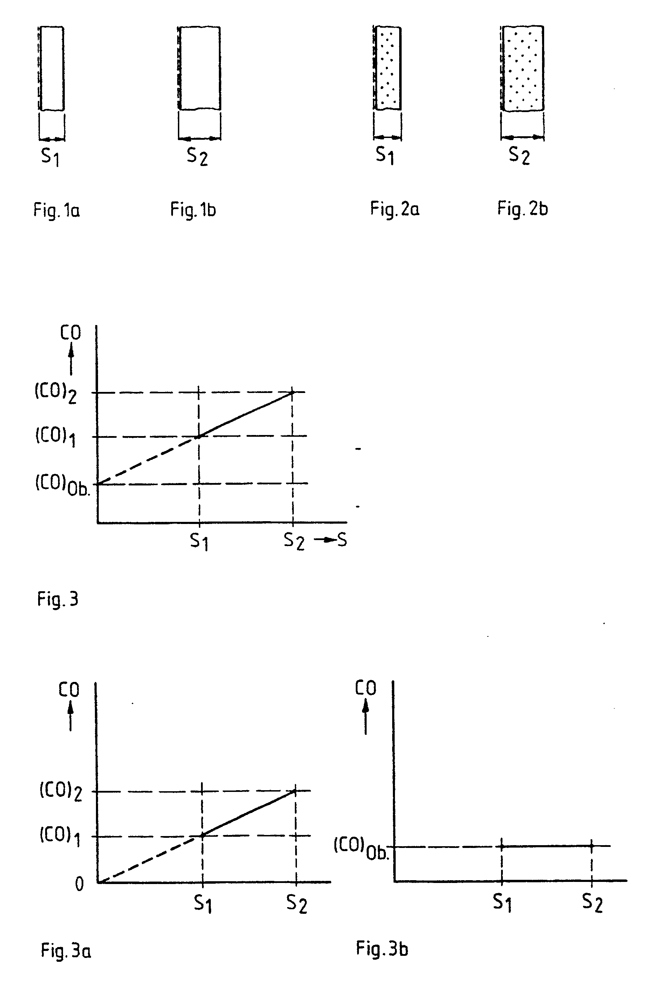

Die der Erfindung zugrundeliegende Hypothese wird zunächst anhand der Fig. 1 bis 3 erläutert.

Es sei s die Dicke bzw. Wandstärke des bei der infrarot-spektrometrischen Messung zu durchstrahlenden Untersuchungskörpers und $s_1$ und $s_2$ jeweils die Dicke an zwei verschieden dicken Meßstellen eines Untersuchungskörpers. Dabei sei $s_2$ signifikant größer als $s_1$. Die Untersuchungskörper der Fig. 1a und 2a mögen dabei die Wandstärke $s_1$ und die Untersuchungskörper der Fig. 1b und 2b die Wandstärke $s_2$ haben. Dabei handelt es sich im Falle der Fig. 1a und 1b vorzugsweise, jedoch nicht notwendigerweise, um verschieden dicke Stellen desselben Untersuchungskörpers. Entsprechendes gilt für die Fig. 2a und 2b.

Die in den Fig. 1a und 1b dargestellten Meßstellen entsprechen Untersuchungskörpern aus Neuware, während die in den Fig. 2a und 2b dargestellten Meßstellen Untersuchungskörpern aus Regenerat entsprechen. In dem Regenerat sind in der Tiefe mit gleichmäßiger Konzentration Carbonyl- bzw. CO-Gruppen verteilt, was in den Fig. 2a und 2b durch eine entsprechende Punktierung angedeutet ist. Diese Punktierung ist in den Fig. 1a und 1b fortgelassen, weil bei der Neuware Freiheit von Carbonyl-Gruppen in der Tiefe vorausgesetzt wird.

An der linken Darstellungsseite der Fig. 1a und 1b sowie 2a und 2b ist ferner eine Strichelung eingezeichnet. Diese soll eine Oberflächenzone des Probenkörpers symbolisieren, deren Tiefe gegenüber der Dicke oder Wandstärke $s_1$ oder $s_2$ des jeweiligen Untersuchungskörpers zu vernachlässigen ist.

Ohne Beschränkung der Allgemeinheit wird dabei lediglich zur Veranschaulichung davon ausgegangen, daß eine solche Oberflächenzone nur an einer Seite des Untersuchungskörpers vorhanden ist. Tatsächlich wird eine solche Oberflächenzone jedoch auf beiden Seiten des Untersuchungskörpers vorhanden sein können, wenn auch meist eine Oberflächenseite in stärkerem Maße der Photo-Oxidation durch Sonneneinstrahlung ausgesetzt sein wird als die andere.

Der Anteil der gemessenen Transmissions-Absorption im Bereich der Carbonyl-Gruppen-Bande, welcher aus der Oberflächenzone stammt, sei $(CO)_{Ob.}$. Dieser Wert ist im Falle formfrischer Untersuchungskörper gleich oder nahezu Null. Er darf, egal, ob es sich um Neuware oder um Ware aus Regenerat handelt, bis zu einem bestimmten Schwellwert ansteigen - der bei höherer Differenzierung auch von der Art des Materials (ob Neuware oder Regenerat oder Sauerstoffhaltigkeit des Regenerats) abhängen kann. Oberhalb des festzulegenden Schwellwertes ist von einer solchen Oberflächensprödigkeit auszugehen, daß die betreffenden Untersuchungskörper als zerstörungsempfindlich auszusortieren sind.

Ferner sei die über die bestimmten Dicken $s_1$ oder $s_2$ insgesamt gemessene (kalibrierte) Transmissions-Absorption im Bereich der Carbonyl-Gruppen-Bande mit $(CO)_1$ bzw. $(CO)_2$ oder, als allgemeine Variable, mit CO bezeichnet. Dann ergibt sich unter Berücksichtigung der Tatsache, daß nach dem Beer-Lambert'schen Gesetz die Zunahme der Carbonyl-Gruppen mit der Dicke proportional zu deren Konzentration sowie zu der Dicke ist, die aus Fig. 3 ersichtliche Beziehung.

Diese läßt sich nach dem Strahlengang durch die nachfolgende Gleichung ausdrücken:

$$\frac{(CO)_2 - (CO)_{Ob.}}{(CO)_1 - (CO)_{Ob.}} = \frac{s_2}{s_1} \qquad . \qquad (1)$$

Aufgelöst nach $(CO)_{Ob.}$ folgt:

$$(CO)_{Ob.} = \frac{s_2 \, (CO)_1 - s_1 \, (CO)_2}{s_2 - s_1} \qquad . \qquad (2)$$

Wenn es sich gemäß Fig. 1a und 1b um einen Untersuchungskörper aus Neuware handelt, gilt folgender Sonderfall gemäß Fig. 3b:

$$(CO)_{Ob.} = (CO)_1 = (CO)_2 \qquad . \qquad (3)$$

Bei nicht gealtertem Untersuchungskörper aus Neuware mit $(CO)_{Ob.} = 0$ liegen in Fig. 3b also auch die Meßwerte bei $s_1$ und $s_2$ auf der Abszisse.

Andernfalls nimmt CO von dem Schwellwert $(CO)_{Ob.}$ linear mit der Dicke s zu. In diesem Fall handelt es sich um einen Untersuchungskörper aus Regenerat gemäß den Fig. 2a und 2b, wobei an den Stellen unterschiedlicher Dicke die gleiche Carbonyl-Gruppen-Konzentration vorausgesetzt wird.

Fig. 3a entspricht dem Meßergebnis an einem Untersuchungskörper aus noch nicht gealtertem Regenerat mit $(CO)Ob. = 0$. In allen Fällen kann der (extrapolierte) Wert von $(CO)_{Ob.}$ als kritischer Wert für die Alterung angesehen werden.

Die Konzentration dieser Carbonyl-Gruppen, also ein für die Art des Regenerats charakteristisches Maß, läßt sich aus der Steilheit, also dem Differentialquotienten, der Meßkurve gemäß Fig. 3 bzw. 3a ermitteln.

Die Erfindung geht davon aus und beruht auf der Annahme, daß Entsprechendes auch gilt, wenn in praxi die Verhältnisse dieser Hypothese nicht in reiner Form vorliegen. Auch dann wird man aus der Form der Absorptionsabhängigkeit von der Dicke im Sinne von Fig. 3 zwischen Neuware und Regenerat, aus dem Differentialquotienten auf den Oxidationsgrad des Regenerats und aus dem extrapolierten Wert $(CO)_{Ob.}$ auf die oberflächliche Oxidation als kritisches Maß der Alterung des Untersuchungskörpers schließen können, im letzteren Falle sogar ganz unabhängig davon, ob es sich um einen Untersuchungskörper aus Neuware oder aus mehr oder minder carbonyl-gruppen-haltigem Regenerat handelt.

Die erwähnten Meßgrößen können daher in bekannter Weise zur Klassifizierung und gegebenenfalls Sortierung nach Neuware oder Regenerat, Art des Regenerats, Alterungszustand und Eignung eines Altkastens für Regeneratgewinnung verwendet werden.

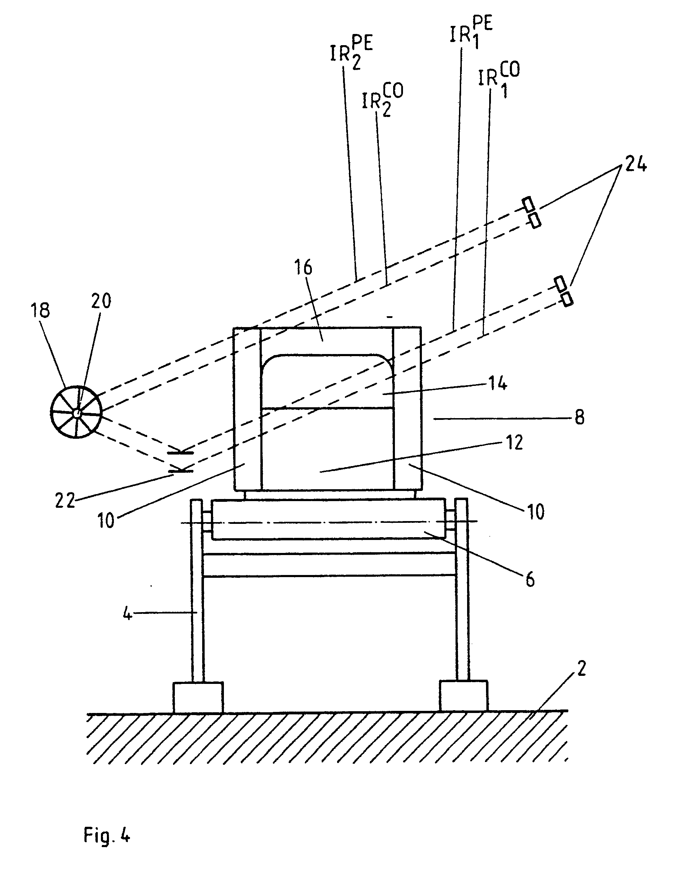

Anhand von Fig. 4 soll nun eine bevorzugte unter vielen möglichen Vorrichtungen zum Ausführen des erfindungsgemäßen Verfahrens beschrieben werden.

Auf einer Basis 2 ist ein Lagergestell 4 einer Rollenbahn 6 aufgestellt, über die Flaschenkästen 8 sukzessive in die dargestellte Meßvorrichtung geführt werden können. Die Rollenbahn 6 ist dabei nur ein Beispiel unter einer Vielzahl möglicher Transferstrecken. Ebenso kommen beispielsweise Bandförderer, Rundförderer usw. in Frage. Auch die Flaschenkästen 8 stellen nur ein bevorzugtes Beispiel unter einer Vielzahl in Frage kommender Untersuchungskörper, insbesondere Spritzgußgroßteilen, dar.

Der Flaschenkasten 8 ist mit verstärkten Eckpfeilern 10 dargestellt, zwischen denen sich Seitenwände erstrecken, die sich aus einem relativ dünnen folienartigen unteren Seitenwandabschnitt 12, einer darüber befindlichen Grifföffnung 14 und einer Griffleiste 16 zusammensetzen. Die Wandstärke der Eckpfeiler 10 sowie der Griffleiste 16 ist etwa um den Faktor zwei oder mehr größer als die Wandstärke des unteren Seitenwandabschnitts 12, dessen Wandstärke typischerweise bei etwa 1,5 cm liegt.

Der Flaschenkasten 8 möge aus Niederdruck-Polyäthylen bzw. HDPE (high density polyethylene) bestehen.

Eine Infrarot-Lichtquelle 18 wird mit zwei Filtern bestückt, von denen der eine nur Infrarotlicht mit Wellenzahlen entsprechend der Carbonyl-Gruppen-Bande und der andere nur Infrarotlicht entsprechend der PE-Bande hindurchläßt. Die entsprechende Filtereinrichtung ist symbolisch mit 20 bezeichnet.

Die beiden Infrarot-Lichtstrahlen verschiedener Wellenlänge werden jeweils in (mindestens) zwei Teilstrahlen aufgeteilt, die durch Stellen verschiedener Dicke des Flaschenkastens 8 hindurchgeleitet werden. Eine solche Strahlaufteilung kann beispielsweise mittels eines halbdurchlässigen Spiegels erfolgen.

Ohne Beschränkung der Allgemeinheit tritt im dargestellten Ausführungsbeispiel der eine Teilstrahl aus der Infrarot-Lichtquelle jeweils geradlinig in den Flaschenkasten 8 ein, während der andere Teilstrahl jeweils durch eine Spiegeleinrichtung 22 zur anderen Meßstelle am Flaschenkasten 8 umgelenkt wird. Über bekannte optische Mittel kann man jedoch auch die Teilstrahlen anders lenken. Die hier gezeigte Anordnung, bei der alle Teilstrahlen parallel zueinander verlaufen, hat jedoch vorrichtungsmäßige Vorteile. So kann man, wie dargestellt, die Infrarot-Lichtquelle 18 mitsamt den Filtern 20 sowie die Spiegeleinrichtung 22 neben der einen Seite des Flaschenkastens 8 und vier Empfänger 24 für jeden Teilstrahl neben der anderen Seite des Flaschenkastens 8, hier zweckmäßig etwas höher, anordnen, so daß trotz des Vorhandenseins von vier Meßstrahlen die Vorrichtung von oben her zugänglich bleibt. Die Infrarotstrahlen IR erhalten dabei den oberen Index CO, wenn ihre Wellenzahlen der Carbonyl-Gruppen-Bande entsprechen, und den oberen Index PE, wenn sie der Polyäthylen-Bande entsprechen.

Mit dem unteren Index 1 bzw. 2 werden ferner entsprechend der vorhergehenden Diskussion anhand der Fig. 1 bis 3 Meßstrahlen bezeichnet, welche durch die dünnere Meßstelle 1 oder die dickere Meßstelle 2 geleitet werden. Als dünnere Meßstelle 1 ist hier der normalerweise auch die Werbefläche darbietende untere Seitenwandabschnitt 12 gewählt, während als dickere Meßstelle entweder die Griffleiste 16 (in einem von einem etwa umlaufenden Rand unabgeschatteten unteren Bereich) oder ein nicht abgeschatteter oberer Endbereich eines Eckpfeilers 10 oder aber ein nicht dargestellter, z.B. flanschartiger, oberer Flaschenrand gewählt ist. Die zeichnerische Darstellung gemäß Fig. 4 paßt auf alle drei genannten Möglichkeiten. Die tatsächlichen Durchstrahlungsstellen an den Meßstellen 1 und 2 sind dabei nicht identisch, jedoch so nahe benachbart, daß gleiche Wandstärken vorausgesetzt werden können. Es ist jedoch auch eine Durchstrahlung an im wesentlichen denselben Meßstellen möglich, solange man noch die einerseits auf die Carbonyl-Gruppen-Bande und die andererseits auf die PE-Bande eingestellten Meßstrahlen gesondert verarbeiten kann.

Bei dem Ausführungsbeispiel treten die Meßstrahlen an der Meßstelle 1 durch den einen unteren Seitenwandabschnitt 12 hindurch und verlassen dann den Flaschenkasten durch die gegenüberliegende Grifföffnung 14, so daß nur eine einmalige Wanddurchstrahlung erfolgt. Die Meßstrahlen an der Meßstelle 2 treten nach Durchstrahlung der ersten Wand direkt nach oben über den Flaschenkasten 8 aus, so daß sie die gegenüberliegende Flaschenkastenwand nicht mehr erreichen. Diese Durchstrahlung jeweils nur einer Wand des Flaschenkastens 8 begründet die Neigung der Meßstrahlen IR schräg von unten nach oben. Man kann jedoch zweckmäßig auch den Strahlengang so abwandeln, daß jeweils zwei gegenüberliegende Wände zugleich von demselben Meßstrahl IR durchstrahlt werden. Dies kann beispielsweise zu einem horizontalen parallelen Strahlengang führen. Die Parallelität der Strahlen ist jedoch keine funktionelle Notwendigkeit, sondern nur manchmal aus Gründen einfachen apparativen Aufbaus zweckmäßig.

Für manche Anwendungsfälle kann es ausreichen, nur die Meßstrahlen $IR^{CO}$ vorzusehen und die Meßstrahlen IR$^{PE}$ ausschaltbar zu machen oder von vornherein nicht vorzusehen. Das gilt insbesondere dann, wenn nur zwischen Flaschenkästen aus Neuware und aus Regenerat unterschieden werden soll. Bei Alterungsmessungen und meist auch bei Messungen des inneren Oxidationsgrades von Regenerat empfiehlt es sich jedoch, die Genauigkeit der Messung durch zusätzliche Verwendung der Meßstrahlen IR$^{PE}$ zu erhöhen. Eine besonders bevorzugte Vorrichtung sieht daher alle vier Meßstrahlen,oder noch weitere Meßstrahlenpaare, vor, von denen einige wahlweise außer Betrieb gesetzt werden können.

Bei Verwendung aller vier Meßstrahlen IR werden die an den vier Empfängern 24 gewonnenen Transmissions-Absorptions-Werte über einen Multiplexer (Abfrager) an einen Analog-Wandler weitergegeben, der die Werte an einen angeschlossenen Computer zur Auswertung und zur Entscheidung weitergibt, ob der untersuchte Flaschenkasten 8 als brauchbar passieren kann, ob er wegen Erreichens einer bestimmten CO-Gruppen-Konzentration an der Oberfläche als zu regenerieren ausgesondert wird oder ob er gar wegen Überschreitens einer maximalen CO-Gruppen-Konzentration an der Oberfläche, insbesondere bei einem Flaschenkasten aus Regenerat, als unbrauchbar und als nicht mehr zu regenerieren ausgesondert werden soll.

Ein mehr- oder gar vielfacher Durchgang durch Wände des Untersuchungskörpers, hier des Flaschenkastens 8, kann im übrigen auch dadurch erfolgen, daß durch entsprechende Spiegeleinrichtungen (einschließlich anderer optischer Umlenkelemente) dieselbe Wand von einem Meßstrahl mehrfach durchquert wird. Hierbei könnte man beispielsweise Sender und Empfänger an derselben Seite des Flaschenkastens und eine Spiegeleinrichtung alternativ über dem Flaschenkasten, hinter Grifföffnungen, oder, was erhöhten konstruktiven Aufwand bedeuten würde, unter taktweisem Einbringen innerhalb des Flaschenkastens anordnen.

Im allgemeinen wird eine einmalige Wanddurchstrahlung ausreichen, zumal sie minimale Reflexionsverluste zeigt. Es ist zweckmäßig, alle Meßstrahlen so zu führen, daß die Facheinteilung eines Flaschenkastens nicht getroffen wird.

## Ansprüche

1. Verfahren zum Bestimmen der Eigenschaften von gleichartigen Kunststoffkörpern, insbesondere aus Polyäthylen oder Polypropylen,
   - ob sie aus unregeneriertem oder regeneriertem Kunststoff bestehen,
   - wie hoch bei regeneriertem Kunststoff der Grad der in der Tiefe verteilten Oxidation ist, und/oder
   - wie hoch der Grad der Oberflächenoxidation ist, unabhängig davon, ob die Kunststoffkörper aus unregeneriertem oder regeneriertem Kunststoff bestehen, dadurch gekennzeichnet, daß die IR-Transmission im Bereich der Carbonylgruppenbande bei mindestens zwei verschiedenen Dicken des Kunststoffs gemessen wird.

2. Verfahren nach Anspruch 1, dadurch gekennzeichnet, daß mindestens zwei Meßstrahlen (IR$^{CO}$) mit Wellenzahlen im Bereich der Carbonylgruppenbande des Kunststoffs ein- oder mehrfach durch unterschiedlich dicke Stellen von Kunststoffkörpern geleitet werden.

3. Verfahren nach Anspruch 1 oder 2, dadurch gekennzeichnet, daß zusätzlich die Dickem durch Messen der IR-Transmission im Bereich einer von der durchstrahlten Kunststoffkonzentration abhängigen, aber von Carbonylgruppen unabhängigen Bande nach dem Beer-Lambert'schen Gesetz bestimmt werden.

4. Verfahren nach Anspruch 3, dadurch gekennzeichnet, daß mindestens zwei Meßstrahlen (IR$^{PE}$) mit Wellenzahlen einer von der durchstrahlten Kunststoffkonzentration des Kunststoffkörpers abhängigen, aber von Carbonylgruppen unabhängigen Bande durch dieselben Stellen der Kunststoffkörper, an denen die IR-Transmissionen im Bereich der Carbonylgruppen-Bande bestimmt werden, oder Stellen entsprechender Dicken der Kunststoffkörper geleitet werden.

5. Verfahren nach einem der Ansprüche 1 bis 4, dadurch gekennzeichnet, daß die bei verschiedenen Dicken vorgenommenen Messungen an Stellen verschiedener Dicke desselben Kunststoffkörpers vorgenommen werden.

6. Verfahren nach einem der Ansprüche 1 bis 5, dadurch gekennzeichnet, daß zugleich mehrere Wände des Kunststoffkörpers durchstrahlt werden.

7. Verwendung des Verfahrens nach einem der Ansprüche 1 bis 6 für die Untersuchung von Spritzgußgroßteilen, vorzugsweise Flaschentransportkästen.

8. Verwendung nach Anspruch 7, dadurch gekennzeichnet, daß bei Flaschenkästen die Meßstrahlen ($IR^{CO}$ und gegebenenfalls $IR^{PE}$) einerseits durch einen normalerweise dem Sonnenlicht frei exponierten Bereich einer Griffleiste, eines Eckpfeilers oder einer oberen Randkante und andererseits durch einen normalerweise dem Sonnenlicht frei exponierten Abschnitt der unteren Seitenwand des Flaschenkastens geleitet werden.

9. Verwendung des Verfahrens nach einem der Ansprüche 1 bis 6 zur Aussortierung nicht mehr weiterverwendbarer Kunststoffkörper.

10. Verwendung des Verfahrens nach einem der Ansprüche 1 bis 6 zur Aussortierung von als Regeneratmaterial geeigneten Kunststoffkörpern.

11. Vorrichtung zur Durchführung des Verfahrens nach einem der Ansprüche 1 bis 6 gemäß der Verwendung nach Anspruch 7 oder 8, gekennzeichnet durch einen Sender (18) zur Aussendung der IR-Strahlen ($IR^{CO}$ und gegebenenfalls $IR^{PE}$) und eine zugehörige Empfangseinrichtung (24), die beidseitig des Spritzgußgroßteils (8) angeordnet sind, und durch eine optische Einrichtung (20,22), welche die Strahlung des Senders in mindestens zwei Meßstrahlen ($IR_1^{CO}$ und $IR_2^{CO}$ und gegebenenfalls $IR_1^{PE}$ und $IR_2^{PE}$) so aufteilt das diese durch unterschiedlich dicke Bereiche des Spritzgußgroßteils geleitet werden und denen gesonderte Empfänger der Empfangseinrichtung (24) zugeordnet sind.

12. Vorrichtung nach Anspruch 11, dadurch gekennzeichnet, daß das Spritzgußgroßteil (8) von oben zugänglich ist.

13. Vorrichtung nach Anspruch 11 oder 12, dadurch gekennzeichnet, daß alle Meßstrahlen (IR) parallel zueinander verlaufen.

14. Vorrichtung nach einem der Ansprüche 11 bis 13, dadurch gekennzeichnet, daß die Meßstrahlen (IR) schräg von unten nach oben verlaufen.

## Claims

1. Method for determining the properties of similar bodies of plastics material, especially of polyethylene or polypropylene,
   - whether they consist of unregenerated or regenerated plastics material,
   - in the case of regenerated plastics material, how great the degree of distributed oxidation at depth, and/or,
   - how great the degree of surface oxidation, regardless of whether the plastics material bodies consist of unregenerated or regenerated plastics material, characterised in that the infra-red transmission in the area of the carbonyl group band is measured at at least two different thicknesses of the plastics material.

2. Method according to claim 1, characterised in that at least two measuring beams ($IR^{CO}$) with wave numbers in the area of the carbonyl group band of the plastics material are directed once or more often through locations of different thicknesses of the bodies of plastics material.

3. Method according to claim 1 or 2, characterised in that additionally the thicknesses are determined according to Beer Lambert's law by measurement of the infra-red transmission in the area of a band dependent on the plastics material concentration traversed by the radiation but independent of carbonyl groups.

4. Method according to claim 3, characterised in that at least two measuring beams ($IR^{PE}$) with wave numbers of a band dependent on the plastics material concentration in the body of plastics material traversed by the radiation, but independent of carbonyl groups, are directed through the same locations of the bodies of plastics material in which the infra-red transmissions in the area of the carbonyl bands are determined or locations of corresponding thickness of the bodies of plastics material.

5. Method according to one of claims 1 to 4, characterised in that the measurements taken at different thicknesses, are taken at locations of different thickness of the same body of plastics material.

6. Method according to one of claims 1 to 5, characterised in that a plurality of walls of the body of plastics material are traversed by the radiation simultaneously.

7. Use of the method according to one of claims 1 to 6 for monitoring large bulk injection mouldings, preferably bottle transportation crates.

8. Use according to claim 7, characterised in that, in the case of bottle crates, the measuring beams ($IR^{CO}$ and if appropriate $IR^{PE}$) are directed, on the one hand through the area of a grip section, a corner post or an upper boundary edge which are usually freely exposed to sunlight, and on the other hand through a section of the lower side wall of the bottle crate which is usually freely exposed to sunlight.

9. Use of the method according to one of claims 1 to 6 for segregating bodies of plastics material that are no longer usable.

10. Use of the method according to one of claims 1 to 6 for segregating bodies of plastics material that are suitable as regeneratable material.

11. Apparatus for carrying out the method according to one of claims 1 to 6 in accordance with the use according to claim 7 or 8, characterised by a sender (18) for sending infra-red beams ($IR^{CO}$ and if appropriate $IR^{PE}$) and a corresponding receiving device (24), which are disposed to both sides of the large bulk injection moulding (8), and by an optical device (20,22) which divides the radiation of the sender into at least two measuring beams ($IR_1^{CO}$ and $IR_2^{CO}$ and if appropriate $IR_1^{P\ E}$ and $IR_2^{P\ E}$) so that these are directed through areas of different thickness of the large bulk injection moulding and are directed towards their allocated receivers of the receiving device (24).

12. Apparatus according to claim 11, characterised in that the large bulk injection moulding (8) is accessible from above.

13. Apparatus according to claim 11 or 12, characterised in that all the measuring beams (IR) extend parallel to each other.

14. Apparatus according to one of claims 1 to 13, characterised in that the measuring beams (IR) extend obliquely from bottom to top.

**Revendications**

1. Procédé pour déterminer les propriétés de corps en matière plastique de même type, en particulier en polyéthylène ou polypropylène, pour savoir
   - s'ils consistent en matière plastique non régénérée ou régénérée,
   - quel est le degré de l'oxydation répartie en profondeur, dans le cas d'une matière plastique régénérée, et/ou
   - quel est le degré d'oxydation superficielle, indépendamment du fait que les corps en matière plastique consistent en matière plastique non régénérée ou régénérée, caractérisé en ce que l'on mesure la transmission IR dans la plage de la bande des groupes carbonyle pour au moins deux épaisseurs différentes de la matière plastique.

2. Procédé selon la revendication 1, caractérisé en ce que l'on dirige une ou plusieurs fois sur des sites d'épaisseur différente des corps en matière plastique au moins deux faisceaux de mesure ($IR^{CO}$) de nombres d'onde dans la plage de la bande des groupes carbonyle de la matière plastique.

3. Procédé selon la revendication 1 ou 2, caractérisé en ce que l'on détermine en outre les épaisseurs selon la loi de Beer-Lambert en mesurant la transmission IR dans la plage d'une bande qui dépend de la concentration de la matière plastique irradiée mais qui ne dépend pas des groupes carbonyle.

4. Procédé selon la revendication 3, caractérisé en ce que l'on dirige au moins deux faisceaux de mesure (IR$^{PE}$) de nombres d'onde d'une bande qui dépend de la concentration de la matière plastique irradiée du corps en matière plastique mais qui ne dépend pas des groupes carbonyle sur les sites des corps en matière plastique au niveau desquels on détermine les transmissions IR dans la plage de la bande des groupes carbonyle, ou sur des sites d'épaisseur correspondante des corps en matière plastique.

5. Procédé selon l'une des revendications 1 à 4, caractérisé en ce que l'on fait les mesures effectuées pour des épaisseurs différentes sur des sites d'épaisseur différente du même corps en matière plastique.

6. Procédé selon l'une des revendications 1 à 5, caractérisé en ce que l'on irradie simultanément plusieurs parois du corps en matière plastique.

7. Utilisation du procédé selon l'une des revendications 1 à 6 pour l'examen de pièces de grande taille moulées par injection, de préférence de casiers à bouteilles.

8. Utilisation selon la revendication 7, caractérisée en ce que, dans le cas des casiers à bouteilles, on dirige les faisceaux de mesure (IR$^{CO}$ et éventuellement IR$^{PE}$) d'une part sur un domaine normalement librement exposé à la lumière solaire d'une barre formant poignée, d'un pilier d'angle ou d'un bord supérieur, et d'autre part sur une section normalement librement exposée à la lumière solaire de la paroi latérale inférieure du casier à bouteilles.

9. Utilisation du procédé selon l'une des revendications 1 à 6 pour retirer les corps en matière plastique qui ne sont plus utilisables.

10. Utilisation du procédé selon l'une des revendications 1 à 6 pour retirer les corps en matière plastique qui conviennent comme matière régénérée.

11. Dispositif pour la mise en oeuvre du procédé selon l'une des revendications 1 à 6 en accord avec l'utilisation selon la revendication 7 ou 8, caractérisé par un émetteur (18) destiné à émettre les faisceaux IR (IR$^{CO}$ et éventuellement IR$^{PE}$) et un dispositif de réception associé (24) qui sont disposés des deux côtés de la pièce de grande taille moulée par injection (8), et par un dispositif optique (20, 22) qui divise le rayonnement de l'émetteur en au moins deux faisceaux de mesure (IR$_1^C$ $^O$ et IR$_2^C$ $^O$ et éventuellement IR$_1^P$ $^E$ et IR$_2^P$ $^E$) de telle façon que ceux-ci sont dirigés sur des domaines d'épaisseur différente de la pièce de grande taille moulée par injection et que des récepteurs séparés du dispositif de réception (24) leur sont associés.

12. Dispositif selon la revendication 11, caractérisé en ce que la pièce de grande taille moulée par injection (8) est accessible par le haut.

13. Dispositif selon la revendication 11 ou 12, caractérisé en ce que tous les faisceaux de mesure (IR) se propagent parallèlement entre eux.

14. Dispositif selon l'une des revendications 11 à 13, caractérisé en ce que les faisceaux de mesure (IR) se propagent obliquement de bas en haut.

Fig. 1a          Fig. 1b          Fig. 2a          Fig. 2b

Fig. 3

Fig. 3a                    Fig. 3b

Fig. 4